# EUROPEAN PATENT SPECIFICATION

(11) **EP 3 678 742 B1**
(45) Date of publication and mention of the grant of the patent: **12.02.2025**
(21) Application number: 18789231.0
(22) Date of filing: 06.09.2018
(51) Int. Cl.: A61Q 15/00, A61K 8/02, A61K 8/19

(54) **DEODORANT COMPOSITION**
DESODORIERENDE ZUSAMMENSETZUNG
COMPOSITION DÉODORANTE

(30) Priority: 06.09.2017 NL 2019490; 13.07.2018 NL 2021304
(43) Date of publication of application: 15.07.2020
(73) Proprietor: NUUD B.V., 1059 CM Amsterdam (NL)
(72) Inventor: VAN SETERS, Martijn Pieter, 1072 HC Amsterdam (NL)
(74) Representative: V.O.
(86) International application number: PCT/NL2018/050572
(87) International publication number: WO 2019/050399

(56) References cited:
- WO-A2-2005/023206
- DE-A1- 102012 215 674

## Description

The invention is in the field of skin care products. The invention is particularly directed to deodorant skin care compositions.

In most cultures, human bodily malodors are considered undesirable and the presence thereof is therefore generally as much as possible limited by applying deodorants. Conventionally, deodorants can be categorized in three categories or deodorant types: antimicrobial deodorants, malodor-masking deodorants, and antiperspirants. These types differ in action and ingredients. The malodor is typically created by bacterial secretion of malodorous compounds such as butyric acid. Since the bacteria proliferate particularly well in moisture secreted by apocrine sweat glands, such as in the moist environment of sweaty armpits, malodors are generally associated with perspiration or sweating. The types of deodorants differ on which stage of the malodor production is acted upon. For instance, odor-masking deodorants act by masking the presence of the malodorous compounds secreted by the bacteria with perfumes, while antiperspirants act by blocking the sweat glands to reduce the perspiration and thus the moist climate in which bacteria thrive. Antimicrobial deodorants act by killing bacteria and/or by inhibiting growth thereof.

Malodor-masking deodorants have the drawbacks that strong odorous compounds such as perfumes are required. This is generally not desired since the odor may not be perceived pleasant by the consumer, the odor may interfere with other perfumes applied, and the deodorant generally has a poor efficacy and a short effective period. In addition, the presence of perfumes may cause skin irritations.

On the other hand, antiperspirants are generally not desirable due to the requirement of aluminum salts, which may also cause skin irritations. Although alternatives for aluminum salts such as sodium bicarbonate (see Henderson Lamb, Journal of Investigative Dermatology, 7 (1946) 131-133) have been suggested, these alternatives also influence the skin condition resulting in sensitive or irritating skin as well, e.g. due to removal of its protective layer. Moreover, perspiration can be regarded as a natural process required for personal well-being, and perspiration inhibition may therefore not be desired.

An example of a antiperspirant composition is for instance described in DE102012215674. The composition comprises a deodorant or antiperspirant active ingredient and at least one oil that is liquid under normal conditions as a carrier.

For convenience of use, it is preferred that a deodorant remains effective for a long period of time, which reduces the required frequency of application. Furthermore, as application of deodorants may coincide with irritation, a reduced required frequency of application may lead to less irritation of the skin. Conventional deodorants typically remain effectiveness for 1 day.

It is an object of the present invention to provide a deodorant skin care composition that does not require daily application thereof without losing its effectiveness. This object may be met by a deodorant that remains on the skin for a prolonged time, while retaining its effect.

A further object of the present invention is to provide a deodorant skin case composition that is based on natural, sustainable components.

Accordingly, the present invention is directed to deodorant skin care composition comprising an oil-based carrier and porous particles as defined in the claims.

The particular composition of the deodorant according to the present invention has a number of advantages; it has a neutral odor, is easy to spread, generates a pleasant feeling during application, is well-tolerated and non-allergenic, provides long-term deodorization, and has non-staining properties. In particular, the provision of long-term deodorization is due to the unique combination of the oil-based carrier and the porous particles comprising silver.

The present inventors realized that sweat itself is odorless and long-term deodorization may also be achieved by a highly effective antimicrobial agent that remains present and effective for a long period.

However, typical antimicrobial agents such as ethanol, quaternary ammonium salts, glyceryl fatty acid esters, such as diglyceryl monolaurate, and sucrose fatty acid esters, such as sucrose monostearate are easily rinsed of the skin and generally do not show long-term deodorization. Triclosan is another well-known antimicrobial agent but is controversial for environmental concerns and is therefore less preferred. This also applies to silver particles of nano size, which have to meet strong safety and health regulations. In contrast, the porous particles comprising metallic silver according to the present invention are preferably of micro size and may remain on the skin for a long time. In addition, due to the porous character of the particles, their size can be large enough to be safe, without compromising the required large surface area for providing long term effectiveness.

The porous particles comprising metallic silver have an antimicrobial effect and limit the proliferation of microorganisms such as bacteria and hence the production of malodor. In a preferred embodiment of the present invention, the porous particles have a mean diameter between 1 and 100 µm, such that the porous particles can be considered porous microparticles. Preferably the porous macroparticles have a mean diameter between 2 and 20 µm, more preferably between 2 and 5 µm. In addition, it is preferred that the porous particles have a mean inner porosity of at least 65%, preferably between 65 and 90%. The defined size ensures the safety of the particles while the porosity provides a high surface area and a connected high effectiveness. As such, the effectiveness of the microparticles is comparable to or even better than silver nanoparticles, without the accompanying health and safety risks that are typically associated with nanoparticles.

Porous particles comprising metallic silver that are particularly suitable for use in the present invention are those commercially available under the tradename MicroSilver BG^{™} which are described in US2007/0081958 and EP1658041. US2007/0081958 and EP1658041.

The porous particles may already be effective at relatively small amounts. Preferably, the porous particles are present in an amount of 0.05 to 5 wt%, preferably 0.1 to 2 wt% such as about 0.1 to 1 wt% based on the total weight of the deodorant skin care composition. It was found that these amounts provide a comparable or even better effectiveness as triclosan as found in typical amounts in conventional deodorant products for example.

The antimicrobial properties of the porous particles originate from the metallic silver therein. Therefore, the porous particles preferably comprise at least 90 wt% metallic silver, more preferably at least 99%, most preferably at least 99.9 wt% based on the total weight of the porous particles. Other materials that may be present in the porous particles are for instances metallic zinc and metallic copper.

Unlike most deodorants in the art which are alcohol-based, the deodorant of the present invention is based on an oil-based carrier. The deodorant of the present invention is in fact preferably essentially free of water and/or alcohol as carrier. It was found that since the present deodorant composition is oil-based, it is essentially waterproof and may remain present on the skin even after the skin has been rinsed or washed with water. In contrast, alcohol-based deodorants are easily removed from the skin by washing or rinsing the skin with water. In the context of the present invention, essentially free or water and/or alcohol typically means that less than 10 wt%, preferably less than 2 wt% of water and/or alcohol is present in the composition.

Unless explicitly indicated otherwise, all amounts of the ingredients described herein are amounts in weight percentages (wt%) based on the total weight of the deodorant skin care composition.

The deodorant composition of the present invention preferably has the formulation of a cream. The cream can directly be applied on the skin, without the involvement of applicators such as rollers, sprayers and the like. After its application, the deodorant cream can be absorbed by the skin.

The oil-based carrier preferably comprises vegetable oil, mineral oil, shea butter, castor oil, cocoa butter, isopropyl myristate, isohexadecane, paraffin, sterol esters, silicone oil or a combination thereof. In a particular embodiment, the oil-based carrier comprises petrolatum, lanolin, paraffin, paraffinum liquidum or a combination thereof. In a particularly preferred embodiment, the oil-based carrier comprises a vegetable oil or a non-animal-based oil. Mineral oils are less preferred for their fossil-based origin. With the term vegetable oil is meant oils, fats and/or waxes, preferably oils, that are extracts from seeds, leaves or other plant-based materials, or based thereon. Particularly preferably vegetable oils include ricinus communis seed oil, prunus amygdalus dulcis oil, copernicia cerifera cera, polyglyceryl-3 diisostearate and/or hydrogenated castor oil. It was found that these components result in an oil-based deodorant cream according to the present invention that has particularly favorable application characteristics, such a spreadability, lack of stickiness and the like. Preferably, the composition comprises at least 70 wt%, preferably at least 75 wt% of the oil-based carrier, based on the total weight of the composition.

The oil-based carrier can fulfill a number of functions, besides its function as carrier. For instance, the oil-based carrier can act as emollient, moisturizer, emulsifier, viscosizer, skin conditioner, and the like. Certain components of the oil-based carrier may have more than one function in the composition. For instance, petrolatum and lanolin, may act as a moisturizer and emollient. Paraffin may act as a viscosizer as well as a skin conditioner. Since paraffin is a fossil-based oil, it may preferably be replaced by one or more vegetable oils. For this reason, the absence of paraffin may accordingly be preferred as well. Some of the vegetable oils such as copernicia cerifera cera, polyglyceryl-3 diisostearate and/or hydrogenated castor oil may serve as viscosizer as well.

To provide a preferred cream viscosity, one or more viscosizers such as clay, non-aqueous and/or non-alcoholic solvents, and the like may be present in the deodorant composition. Preferably, the composition comprises about 10 wt% of one or more viscosizers such as a combination of one or more clays (*e.g.* stearalkonium bentonite) and one or more solvents (*e.g.* propylene carbonate). It is particularly preferred that the viscosizers are non-animal-based and/or non-fossil-based, e.g. vegetable-based and/or clay-based. For instance, stearalkonium bentonite is a combination of the natural occurring minerals stearalkonium hectorite and bentonite clay. Propylene carbonate is an example of a suitable non-aqueous, non-alcoholic solvent. Propylene carbonate is a particular preferred non-aqueous solvent since it is considered a green, sustainable solvent as carbon dioxide is consumed during the preparation of the solvent.

In a preferred embodiment, the deodorant comprises 2-8, preferably 4-6 wt% stearalkonium bentonite, 0.5-5 preferably, 1-3 wt% propylene carbonate, and/or 0.5-2 wt% paraffin (if present). In a more preferred embodiment, the deodorant comprises 2-8 wt% stearalkonium bentonite and 0.5-5 wt% propylene carbonate.

Further, although the deodorant is typically essentially free of hydrophilic ingredients such as water and/or alcohol, it may be preferred that the deodorant composition comprises components that are generally known as an emulsifier for a homogenous composition. Suitable emulsifiers are polymeric neutral surfactants such as laureth-4. In a preferred embodiment, the deodorant comprises about 5 wt% emulsifier, for instance 3-7 wt% laureth-4. Since laureth-4 is associated with skin irritation as well, it may also be preferred that the deodorant is free of laureth-4. Suitable alternative emulsifiers are vegetable-based, sustainable emulsifiers such as polyglyceryl-3 diisostearate and/or hydrogenated castor oil. Suitable amounts of polyglyceryl-3 diisostearate and hydrogenated castor oil are about 1 to 6 wt% each.

In a particularly preferred embodiment, the deodorant is free of animal-based components and/or fossil-based components such as mineral oils. As such, the deodorant can be considered vegan and natural.

Preferably, the deodorant of the present invention is based on natural and sustainable ingredients. The natural and sustainable origin of the ingredients can not only be contributed to their natural sources, but also from the manner of which the ingredients are obtained from the natural sources. To this end, in the art, differentiation is made between certified sustainable ingredients and non-certified ingredients. A certified sustainable ingredient has a lower or absent contribution on the environment due to greenhouse gas emission, deforestation, pollution, soil erosion and the like. As such, in the embodiments wherein the present deodorant composition comprises certified sustainable ingredients, the deodorant has a lower negatieve environmental impact.

In a preferred embodiment, more than 75 wt% of the deodorant comprises certified sustainable ingredients. Even more preferable, more than 90 wt% of the deodorant comprises certified sustainable ingredients. Examples of acceptable certificates in the context of the present invention include the international COSMOS-standard certificate and/or the ECOCERT certificate, the latter certifying activities according to (EC) Regulations 834/2007, 889/2008 and 1235/2008 relative to organic farming in the European Union, the North American NOP (National Organic Program) Standard, the Japanese JAS (Japanese Agricultural Standard), the Roundtable on Sustainable Palm Oil (RSPO) certification and more. In a typical preferred embodiment, the oil-based carrier consists essentially of certified ingredients.

The application characteristics may also be favorable if the oil-based carrier comprises caprilic/capryc trygliceride. The caprilic/capryc trygliceride mixture generally has an emollient function. The caprylic/capric triglyceride is preferably present in an amount of more than 15 wt%, more preferably more than 20 wt%, most preferably 25 to 35 wt%.

Favorable product properties are also obtained if the oil-based carrier comprises petrolatum in an amount of more than 15 wt%, more preferably more than 20 wt%, most preferably 25 to 35 wt%. It was found that excellent product properties are also obtained the oil-based carrier comprises both caprilic/capryc trygliceride and petrolatum, preferably in about the same amounts such as about 30 wt%. However, since petrolatum is fossil-based, its absence may also be preferred. The emollient function can also suitable be fulfilled by ricinus communis seed oil, which is preferably present in an amount of 20 to 35 wt%.

In a particular embodiment, the deodorant comprises more than one emollient. Favorable skin care properties are obtained if the deodorant comprises at least four emollient ingredients, for instance caprilic/capryc trygliceride, petrolatum, paraffinum liquidum and lanolin. Paraffinum liquidum and lanolin can be present in the deodorant in 2-8 wt% and 1-6 wt% respectively. Alternatively, and preferably petrolatum, paraffinum liquidum and lanolin can individually or all be replaced by copernicia cerifera cera such that vegetable-based emollients can be applied. Copernicia cerifera cera may be present in an mount of 1 to 6 wt%.

The antimicrobial activity and odor limiting deodorant skin care composition of the present invention is generally sufficiently effective such that masking of bodily odors by the addition of perfume is not required. In fact, in a preferred embodiment, the composition has a neutral or absent odor such that the deodorant can be applied together with the user's preferred perfume. Accordingly, the deodorant skin care composition is preferably essentially free of perfume. In the context of the present invention, essentially free or perfume typically means that less than 0.1 wt%, preferably less than 0.01 wt% of perfume is present in the composition, based on the total weight of the composition.

Although masking bodily odors is not required, it may be that the deodorant skin care composition of the present invention comprises an odorous composition. Said odorous composition may primarily serve to signal the applicant that the deodorant has been applied to the skin and does typically not serve to mask any unpleasant bodily odors. Accordingly, its concentration may be lower than is typical for convention deodorant products that comprising a masking perfume. In general, a concentration of the odorous composition such that the deodorant skin care composition is detectible by the human nose after application onto the skin is preferred. Particularly suitable odorous compositions in this respect include essential oils such as hydrophobic liquids containing volatile aroma compounds from plants.

Although not required, it may be preferred that the deodorant composition has moisture-absorbing properties. Moisture absorbance typically not only limits malodor by lessening the suitability of the environment for microbial proliferation, but also contributes to a more pleasant (dry) feeling of the user. Thus preferably, the composition comprises one or more moisture-absorbing agents such as mineral clays. In particular, kaolin has shown preferable properties. The moisture-absorbing agent is preferably present in an amount between 1 and 10 wt%, more preferably between 2 and 8 wt%, most preferably about 5 wt%.

In the art, deodorants are known with antiperspirant properties due to ingredients such as aluminum-based or aluminum-zirconium-based agents that can inhibit sweat production by blocking the eccrine and/or apocrine glands. Although such agents can remain in the duct of the glands for several days (e.g. 7-14 days), they are associated with a number of drawbacks. For instance, there are concerns that inhalation of zirconium-containing agents may result in inflammation reactions. In a preferred embodiment, the present deodorant composition is therefore free of antiperspirant agents such as aluminum-based or aluminum-zirconium-based agents. In addition, it is preferred that the deodorant is free of alternatives thereof such a sodium bicarbonate as well.

It was found that the combination of an oil-based carrier, the porous particles and optionally one or more viscosizers such as a combination of a clay and a non-aqueous, non-alcoholic solvent (e.g. stearalkonium bentonite and propylene carbonate) as such may provide the desired long-term deodorization. Therefore, preferably the deodorant comprises at least the oil-based carrier, the porous particles and one or more viscosizers.

Deodorants without antiperspirant properties do not intervene in natural processes of the user and are therefore considered purely cosmetic.

For an additional protection of the skin, the deodorant skin care composition further comprises zinc oxide which is present in an amount of 5-25 wt%, based on the total weight of the deodorant skin care composition. Zinc oxide is preferably present in an amount of more than 5 wt%, more preferably more than 10 wt%, most preferably about 18 wt% based on the total weight of the deodorant skin care composition. A suitable amount of zinc is thus for example in the range of 8 to 25 wt%. Zinc oxide also has antimicrobial properties and has the additional advantage that it can bind to the odorous chemicals and form odorless complexes with these materials.

The long-term deodorizing properties of the deodorant enable that the consecutive application of the deodorant can be several days apart. A further aspect of the present invention is accordingly a method for applying the deodorant skin care composition, comprising applying the deodorant skin care composition to the skin, wherein the time between two consecutive applications is at least 2 days, preferably at least 3 days, more preferably at least 4 days, most preferably at least 6 days.

The time period during which the deodorant is effective can be assessed by standardized Sniff Test based on the Standard Guide for Sensory Evaluation of Axillary Deodorancy, ASTM E1207-14, wherein an expert panel of odor judges or sniffers trained according to DIN EN 13725: 2003 can carry out these assessments. Such as test can for instance be carried out by proDERM Institute for Applied Dermatological Research GmbH, Schenefeld-Hamburg Germany (see also SOFW Journal 144 (2018) 22-28). It was found that the deodorant comprising the oil-based carrier and porous particles that comprise metallic silver has a long time period of effectiveness, for instance several days. Accordingly, the deodorant composition of the present invention has a time period of effectiveness of preferably at least 24 hours, more preferably at least 48 hours, more preferably at least 60 hours, even more preferably at least 72 hours, most preferably at least 96 hours or 4 days.

The invention may be illustrated with the following non-limiting example.

### EXAMPLE 1

A deodorant skin care composition is provided with the following ingredients:

| **Ingredient (INCI Name)** | **Amount in wt%** | **Role of the ingredient** |
|---|---|---|
| Caprilic/capryc trygliceride | 28 to 32 | Emollient |
| Petrolatum | 28 to 32 | Emollient |
| Zinc Oxide | 10 to 15 | Skin protective |
| Stearalkonium bentonite | 5 | Viscosizer |
| Laureth-4 | 4 to 6 | Emulsifier |
| Kaolin | 3 to 6 | Moisture absorbing powder |
| Paraffinum liquidum | 3 to 6 | Emollient |
| Lanolin | 2 to 5 | Emollient |
| Propylene Carbonate | 2 | Viscosizer |
| Paraffin | 1 | Viscosizer; skin conditioner |
| Porous silver microparticles (CI 77820) | 0.1 to 1 | Antimicrobial |

The nomenclature of the above ingredients (including the CI 77820 nomenclature) is in accordance with the International Nomenclature of Cosmetic Ingredients (INCI).

The deodorant skin care composition demonstrated good effectiveness in the treatment of bodily malodor when applied to the skin, even after the skin was rinsed with water.

### EXAMPLE 2

An antibacterial deodorant skin care composition is provided with the following non-animal-based (*i.e.* vegan) ingredients:

| **Ingredient (INCI Name)** | **Amount in wt%** | **Role of the ingredient** |
|---|---|---|
| Caprilic/capryc trygliceride³ | 24 to 28 | Emollient |
| Ricinus communis seed oil^{1,2} | 24 to 28 | Emollient |
| Zinc Oxide | 15 to 22 | Skin protective |
| Prunus amygdalus dulcis oil¹ | 15 to 20 | Emollient |
| Stearalkonium bentonite | 4 to 6 | Viscosizer |
| Polyglyceryl-3 diisostearate^{1,2} | 2 to 4 | Emulsifier |
| Hydrogenated castor oil^{1,2} | 1 to 4 | Emulsifier |
| Copernicia cerifera cera^{1,2} | 1 to 4 | Emollient |
| Propylene Carbonate | 1 to 3 | Viscosizer |
| Porous silver microparticles (CI 77820)1:2 | 0.1 to 1 | Antimicrobial |

| | | |
|---|---|---|
| ¹ COSMOS certified; ² ECOCERT certified; ³ RSPO certified | | |

The nomenclature of the above ingredients (including the CI 77820 nomenclature) is in accordance with the International Nomenclature of Cosmetic Ingredients (INCI).

The deodorant skin care composition demonstrated good effectiveness in the treatment of bodily malodor when applied to the skin, even after the skin was rinsed with water.

### EXAMPLE 3

A panel consisting of 3814 persons having used the deodorant according to Example 1, provided the following data in a questionary.

### How often did you apply Nuud?

| Every day | 27.87% |
|---|---|
| Every other day | 26.14% |
| Every three days | 28.4% |
| Every four days | 9.81% |
| Every five days | 3.64% |
| Every six days | 1.36% |
| Once a week | 2.78% |

### How soft (i.e. non-irritating) is the deodorant for the skin?

| | |
|---|---|
| ★ | 1.39% |
| ★ ★ | 1.28% |
| ★★★ | 7.11% |
| ★ ★ ★ ★ | 22.36% |
| ★★★★★ | 67.86% |

### How neutral (i.e. non-odorous) is the odor of the deodorant?

| | |
|---|---|
| ★ | 1.31% |
| ★ ★ | 1.44% |
| ★★★ | 6.03% |
| ★ ★ ★ ★ | 15.39% |
| ★★★★★ | 75.83% |

## Claims

1. Deodorant skin care composition comprising an oil-based carrier, zinc oxide, porous particles that comprise metallic silver, wherein the zinc oxide is present in an amount of 5 to 25 wt% based on the total weight of the deodorant skin care composition.

2. Deodorant skin care composition according to claim 1, having the form of a cream.

3. Deodorant skin care composition according to any of the previous claims, wherein the porous particles have a mean diameter between 1 and 100 µm, preferably between 2 to 20 µm, more preferably between 2 to 5 µm.

4. Deodorant skin care composition according to any of the previous claims, wherein the porous particles have a mean inner porosity of at least 65%, preferably between 65 and 90%.

5. Deodorant skin care composition according to any of the previous claims, wherein the porous particles are present in an amount of 0.05 to 5 wt%, preferably 0.1 to 2 wt%, more preferably 0.1 to 1 wt% based on the total weight of the deodorant skin care composition.

6. Deodorant skin care composition according to any of the previous claims having an effectiveness of at least 48 hours, preferably at least 60 hours, more preferably at least 72 hours, most preferably at least 96 hours or 4 days as determined according to ASTM E1207-14.

7. Deodorant skin care composition according to any of the previous claims, wherein the oil-based carrier comprises vegetable oil, mineral oil, sterol ester, silicone oil or a combination thereof, preferably a vegetable oil, more preferably as vegetable oil selected from the group consisting of ricinus communis seed oil, prunus amygdalus dulcis oil, copernicia cerifera cera, polyglyceryl-3 diisostearate, and hydrogenated castor oil, or combinations thereof.

8. Deodorant skin care composition according to any of the previous claims, further comprising caprylic/capric triglyceride, preferably in an amount of more than 15 wt%, more preferably more than 20 wt% based on the total weight of the deodorant skin care composition.

9. Deodorant skin care composition according to any of the previous claims, further comprising one or more viscosizers comprising a combination of a clay and a non-aqueous, non-alcoholic solvent, preferably wherein the clay comprises a bentonite such as stearalkonium bentonite and/or wherein the non-aqueous, non-alcoholic solvent comprises propylene carbonate.

10. Deodorant skin care composition according to any of the previous claims that is essentially free of water and/or alcohol.

11. Deodorant skin care composition according to any of the previous claims that is essentially free of perfume.

12. Deodorant skin care composition according to any of claims 1-10 comprising an odorous composition, preferably an odorous composition comprising one or more essential oils.

13. Deodorant skin care composition according to any of the previous claims comprising one or more, preferably all of the following ingredients in amounts as indicated between the brackets: caprilic/capric triglyceride (20-35 wt%), ricinus communis seed oil (20-35 wt%), zinc oxide (8-25 wt%), prunus amygdalus dulcis oil (10 to 25 wt%), stearalkonium bentonite (2 to 8, preferably 4-6 wt%), polyglyceryl-3 diisostearate (1 to 6 wt%), hydrogenated castor oil (1 to 6 wt%), copernicia cerfifera cera (1 to 6 wt%) and propylene carbonate (0.5-5 wt%), together with the porous particles that comprise metallic silver that is present in an amount of 0.05-5 wt%.

14. Deodorant skin care composition according to any of the previous claims consisting of the following ingredients in the indicated amounts:
| **Ingredient (INCI Name)** | **Amount in wt%** |
|---|---|
| Caprilic/capryc trygliceride | 24 to 28 |
| Ricinus communis seed oil | 24 to 28 |
| Zinc Oxide | 15 to 22 |
| Prunus amygdalus dulcis oil | 15 to 20 |
| Stearalkonium bentonite | 4 to 6 |
| Polyglyceryl-3 diisostearate | 2 to 4 |
| Hydrogenated castor oil | 1 to 4 |
| Copernicia cerifera cera | 1 to 4 |
| Propylene Carbonate | 1 to 3 |
| Porous silver microparticles | 0.1 to 1 |

15. Non-therapeutic use of the deodorant skin care composition according to any of the previous claims in the non-therapeutic treatment of bodily malodor, said treatment comprising applying the deodorant skin care composition to the skin, wherein the time between two consecutive applications is at least 2 days, preferably at least 3 days, more preferably at least 4 days, most preferably at least 6 days.

16. Non-therapeutic method for applying the deodorant skin care composition according to any of claims 1-14, comprising applying the deodorant skin care composition to the skin, wherein the time between two consecutive applications is at least 2 days, preferably at least 3 days, more preferably at least 4 days, most preferably at least 6 days.

## Patentansprüche

1. Desodorierende Hautpflegezusammensetzung, umfassend einen Ölbasierten Träger, Zinkoxid, poröse Partikel, die metallisches Silber umfassen, wobei das Zinkoxid in einer Menge von 5 bis 25 Gew.-%, bezogen auf das Gesamtgewicht der desodorierenden Hautpflegezusammensetzung, vorhanden ist.

2. Desodorierende Hautpflegezusammensetzung nach Anspruch 1 in Form einer Creme.

3. Desodorierende Hautpflegezusammensetzung nach einem der vorstehenden Ansprüche, wobei die porösen Partikel einen mittleren Durchmesser zwischen 1 und 100 µm, vorzugsweise zwischen 2 und 20 µm, besonders bevorzugt zwischen 2 und 5 µm aufweisen.

4. Desodorierende Hautpflegezusammensetzung nach einem der vorstehenden Ansprüche, wobei die porösen Partikel eine mittlere innere Porosität von mindestens 65 %, vorzugsweise zwischen 65 und 90 %, haben.

5. Desodorierende Hautpflegezusammensetzung nach einem der vorstehenden Ansprüche, wobei die porösen Partikel in einer Menge von 0,05 bis 5 Gew.-%, vorzugsweise 0,1 bis 2 Gew.-%, stärker bevorzugt 0,1 bis 1 Gew.-%, bezogen auf das Gesamtgewicht der desodorierenden Hautpflegezusammensetzung, vorhanden sind.

6. Desodorierende Hautpflegezusammensetzung nach einem der vorstehenden Ansprüche mit einer Wirksamkeit von mindestens 48 Stunden, vorzugsweise mindestens 60 Stunden, stärker bevorzugt mindestens 72 Stunden, am stärksten bevorzugt mindestens 96 Stunden oder 4 Tagen, wie gemäß ASTM E1207-14 bestimmt.

7. Desodorierende Hautpflegezusammensetzung nach einem der vorstehenden Ansprüche, wobei der Öl-basierte Träger Pflanzenöl, Mineralöl, Sterolester, Silikonöl oder eine Kombination davon umfasst, vorzugsweise ein Pflanzenöl, stärker bevorzugt als Pflanzenöl ausgewählt aus der Gruppe bestehend aus Rizinusöl-Samenöl *(Ricinus communis),* Mandelöl (*Prunus Amygdalus Dulcis*), Carnaubawachs (*Copernicia cerfifera cera*)*,* Polyglyceryl-3-diisostearat und hydriertem Rizinusöl oder Kombinationen davon.

8. Desodorierende Hautpflegezusammensetzung nach einem der vorstehenden Ansprüche, ferner umfassend Capryl-/Caprinsäuretriglycerid, vorzugsweise in einer Menge von mehr als 15 Gew.-%, stärker bevorzugt mehr als 20 Gew.-%, bezogen auf das Gesamtgewicht der desodorierenden Hautpflegezusammensetzung.

9. Desodorierende Hautpflegezusammensetzung nach einem der vorstehenden Ansprüche, ferner umfassend ein oder mehrere Viskosisatoren, umfassend eine Kombination aus einem Ton und einem nicht-wässrigen, nicht-alkoholischen Lösungsmittel, wobei der Ton vorzugsweise einen Bentonit wie Stearalkoniumbentonit umfasst und/oder wobei das nicht-wässrige, nicht-alkoholische Lösungsmittel Propylencarbonat umfasst.

10. Desodorierende Hautpflegezusammensetzung nach einem der vorstehenden Ansprüche, die im Wesentlichen frei von Wasser und/oder Alkohol ist.

11. Desodorierende Hautpflegezusammensetzung nach einem der vorstehenden Ansprüche, die im Wesentlichen frei von Parfüm ist.

12. Desodorierende Hautpflegezusammensetzung nach einem der Ansprüche 1 bis 10, umfassend eine duftende Zusammensetzung, vorzugsweise eine duftende Zusammensetzung, umfassend ein oder mehrere ätherische Öle.

13. Desodorierende Hautpflegezusammensetzung nach einem der vorstehenden Ansprüche, umfassend einen oder mehrere, vorzugsweise alle der folgenden Inhaltsstoffe in den in Klammern angegebenen Mengen: Caprylsäure/Caprinsäure-Triglycerid (20-35 Gew.-%), Rizinus-Samenöl *(Ricinus communis)* (20-35 Gew.-%), Zinkoxid (8-25 Gew.-%), Mandelöl (*Prunus Amygdalus Dulcis*) (10 bis 25 Gew.-%), Stearalkoniumbentonit (2 bis 8, vorzugsweise 4 bis 6 Gew.-%), Polyglyceryl-3-diisostearat (1 bis 6 Gew.-%), hydriertes Rizinusöl (1 bis 6 Gew.-%), Carnaubawachs (*Copernicia cerfifera cera*) (1 bis 6 Gew.-%) und Propylencarbonat (0,5 bis 5 Gew.-%) zusammen mit den porösen Partikeln, die metallisches Silber umfassen, das in einer Menge von 0,05 bis 5 Gew.-% vorhanden ist.

14. Desodorierende Hautpflegezusammensetzung nach einem der vorstehenden Ansprüche, bestehend aus den folgenden Bestandteilen in den angegebenen Mengen:
| **Inhaltsstoff (INCI-Bezeichnung)** | **Menge in Gew.-%** |
|---|---|
| Caprylsäure/Caprinsäure-Tryglicerid | 24 bis 28 |
| Rizinusöl-Samenöl (*Ricinus communis*) | 24 bis 28 |
| Zinkoxid | 15 bis 22 |
| Mandelöl (*Prunus Amygdalus Dulcis*) | 15 bis 20 |
| Stearalkonium-Bentonit | 4 bis 6 |
| Polyglyceryl-3-diisostearat | 2 bis 4 |
| Hydriertes Rizinusöl | 1 bis 4 |
| Carnaubawachs (*Copernicia cerfifera cera*) | 1 bis 4 |
| Propylencarbonat | 1 bis 3 |
| Poröse Silber-Mikropartikel | 0,1 bis 1 |

15. Nicht-therapeutische Verwendung der desodorierenden Hautpflegezusammensetzung nach einem der vorstehenden Ansprüche bei der nicht-therapeutischen Behandlung von Körpergeruch, wobei die Behandlung das Auftragen der desodorierenden Hautpflegezusammensetzung auf die Haut umfasst, wobei die Zeit zwischen zwei aufeinanderfolgenden Anwendungen mindestens 2 Tage, vorzugsweise mindestens 3 Tage, stärker bevorzugt mindestens 4 Tage und am stärksten bevorzugt mindestens 6 Tage ist.

16. Nicht-therapeutische Methode zur Anwendung der desodorierenden Hautpflegezusammensetzung nach einem der Ansprüche 1 bis 14, umfassend das Auftragen der desodorierenden Hautpflegezusammensetzung auf die Haut, wobei die Zeit zwischen zwei aufeinanderfolgenden Anwendungen mindestens 2 Tage, vorzugsweise mindestens 3 Tage, stärker bevorzugt mindestens 4 Tage und am stärksten bevorzugt mindestens 6 Tage ist.

## Revendications

1. Composition déodorante pour soins de la peau comprenant un support à base d'huile, d'oxyde de zinc, de particules poreuses qui comprennent de l'argent métallique, l'oxyde de zinc étant présent en une quantité de 5 à 25 % en poids par rapport au poids total de la composition déodorante pour soins de la peau.

2. Composition déodorante pour soins de la peau selon la revendication 1, se présentant sous la forme d'une crème.

3. Composition déodorante pour soins de la peau selon l'une quelconque des revendications précédentes, dans laquelle les particules poreuses ont un diamètre moyen compris entre 1 et 100 µm, de préférence entre 2 et 20 µm, plus préférablement entre 2 et 5 µm.

4. Composition déodorante pour soins de la peau, selon l'une quelconque des revendications précédentes, dans laquelle les particules poreuses ont une porosité interne moyenne d'au moins 65 %, de préférence comprise entre 65 et 90 %.

5. Composition déodorante pour soins de la peau selon l'une quelconque des revendications précédentes, dans laquelle les particules poreuses sont présentes en une quantité de 0,05 à 5 % en poids, de préférence de 0,1 à 2 % en poids, plus préférablement de 0,1 à 1 % en poids par rapport au poids total de la composition déodorante pour soins de la peau.

6. Composition déodorante pour soins de la peau, selon l'une quelconque des revendications précédentes, ayant une efficacité d'au moins 48 heures, de préférence d'au moins 60 heures, plus préférablement d'au moins 72 heures, le plus préférablement d'au moins 96 heures ou 4 jours telle que déterminée selon la norme ASTM E1207-14.

7. Composition déodorante pour soins de la peau selon l'une quelconque des revendications précédentes, dans laquelle le support à base d'huile comprend de l'huile végétale, de l'huile minérale, de l'ester de stérol, de l'huile de silicone ou une combinaison de celles-ci, de préférence une huile végétale, plus préférablement une huile végétale choisie dans le groupe constitué par l'huile de graines de ricin commun, (*Ricinus communis*), l'huile d'amandes douces, (*prunus amygdalus dulcis*), cire de carnauba, (*copernicia cerifera cera*), le diisostéarate de polyglycéryle-3 et l'huile de ricin hydrogénée, ou des combinaisons de celles-ci.

8. Composition déodorante pour soins de la peau selon l'une quelconque des revendications précédentes, comprenant en outre du triglycéride caprylique/caprique, de préférence en une quantité supérieure à 15 % en poids, plus préférablement supérieure à 20 % en poids sur la base du poids total de la composition déodorante de soins pour la peau.

9. Composition déodorante pour soins de la peau selon l'une quelconque des revendications précédentes, comprenant en outre un ou plusieurs agents viscosants comprenant une combinaison d'une argile et d'un solvant non aqueux et non alcoolique, de préférence dans laquelle l'argile comprend une bentonite telle que la bentonite de stéaralkonium et/ou dans laquelle le solvant non aqueux et non alcoolique comprend du carbonate de propylène.

10. Composition déodorante pour soins de la peau selon l'une quelconque des revendications précédentes, qui est essentiellement exempte d'eau et/ou d'alcool.

11. Composition déodorante pour soins de la peau selon l'une quelconque des revendications précédentes, qui est essentiellement exempte de parfum.

12. Composition déodorante pour soins de la peau selon l'une quelconque des revendications 1 à 10, comprenant une composition odorante, de préférence une composition odorante comprenant une ou plusieurs huiles essentielles.

13. Composition déodorante pour soins de la peau selon l'une quelconque des revendications précédentes, comprenant un ou plusieurs des ingrédients suivants, de préférence tous les ingrédients suivants, dans les quantités indiquées entre parenthèses: triglycéride caprylique/caprique (20 à 35 % en poids), huile de graines de ricinus communis (*ricinus communis seed oil* ; 20 à 35 % en poids), oxyde de zinc (8 à 25 % en poids), l'huile d'amandes douces (*prunus amygdalus dulcis oil*; 10 à 25 % en poids), bentonite de stéaralkonium (2 à 8, de préférence 4 à 6 % en poids), diisostéarate de polyglycéryle-3 (1 à 6 % en poids), huile de ricin hydrogénée (1 à 6 % en poids), cire de carnauba (*copernicia cerfifera cera*; 1 à 6 % en poids) et carbonate de propylène (0,5 à 5 % en poids), ainsi que les particules poreuses, qui comprennent de l'argent métallique, présentes en une quantité de 0,05 à 5 % en poids.

14. Composition déodorante pour soins de la peau selon l'une quelconque des revendications précédentes, constituée des ingrédients suivants dans les quantités indiquées :
| **Ingrédient (nom INCI)** | **Qté en % poids** |
|---|---|
| Triglycéride caprylique/caprique | 24 to 28 |
| Huile de graines de Ricin | 24 to 28 |
| Oxyde de zinc | 15 to 22 |
| Huile de Prunus amygdalus dulcis | 15 to 20 |
| Bentonite de stéaralkonium | 4 to 6 |
| Diisostéarate de polyglycéryl-3 | 2 to 4 |
| Huile de ricin hydrogénée | 1 to 4 |
| Cera de Copernicia cerifera | 1 to 4 |
| Carbonate de propylène | 1 to 3 |
| Microparticules d'argent poreuses | 0.1 to 1 |

15. Utilisation non thérapeutique de la composition déodorante pour soins de la peau selon l'une quelconque des revendications précédentes dans le traitement non thérapeutique des mauvaises odeurs corporelles, ledit traitement comprenant l'application de la composition déodorante pour soins de la peau, le temps entre deux applications consécutives étant d'au moins 2 jours, de préférence d'au moins 3 jours, de préférence encore d'au moins 4 jours, de préférence encore d'au moins 6 jours.

16. Procédé non thérapeutique d'application de la composition déodorante pour soins de la peau selon l'une quelconque des revendications 1 à 14, comprenant l'application de la composition déodorante pour soins de la peau, le temps entre deux applications consécutives étant d'au moins 2 jours, de préférence d'au moins 3 jours, de préférence encore d'au moins 4 jours, de préférence encore d'au moins 6 jours.
